# EUROPÄISCHE PATENTSCHRIFT

(11) **EP 1 325 471 B1**
(45) Veröffentlichungstag und Bekanntmachung des Hinweises auf die Patenterteilung: **04.05.2011**
(21) Anmeldenummer: 01986787.8
(22) Anmeldetag: 11.10.2001
(51) Int. Cl.: G06T 11/00

(54) **DARSTELLUNG EINES OBJEKTES MITTELS EINER DURCHSTRAHLUNG SOWIE REKONSTRUKTION UNTER VERWENDUNG VON SIMULIERTEN DURCHSTRAHLUNGSDATEN**
METHOD AND DEVICE FOR REPRESENTING AN OBJECT BY MEANS OF AN IRRADIATION, AND FOR RECONSTRUCTING SAID OBJECT
PROCEDE ET DISPOSITIF POUR LA REPRESENTATION D'UN OBJET AU MOYEN D'UNE IRRADIATION AINSI QUE POUR LA RECONSTRUCTION

(30) Priorität: 11.10.2000 DE 10050250
(43) Veröffentlichungstag der Anmeldung: 09.07.2003
(73) Patentinhaber: Fraunhofer-Gesellschaft zur Förderung der angewandten Forschung e.V., 80686 München (DE)
(72) Erfinder: HANKE, Randolf, 90617 Puschendorf (DE); SCHRÖPFER, Stefan, 90522 Oberasbach (DE); GERHÄUSER, Heinz, 91344 Waischenfeld (DE); PAULUS, Dietrich, 91074 Herzogenaurach (DE)
(74) Vertreter: Schoppe, Fritz
(86) Internationale Anmeldenummer: PCT/EP2001/011796
(87) Internationale Veröffentlichungsnummer: WO 2002/031767

(56) Entgegenhaltungen:
- US-A- 4 920 491
- US-A- 6 061 420
- US-A- 6 101 236
- DUVAUCHELLE P ET AL: "A computer code to simulate X-ray imaging techniques" NUCLEAR INSTRUMENTS & METHODS IN PHYSICS RESEARCH, SECTION B (BEAM INTERACTIONS WITH MATERIALS AND ATOMS), SEPT. 2000, ELSEVIER, NETHERLANDS, Bd. 170, Nr. 1-2, Seiten 245-258, XP004216329 ISSN: 0168-583X
- KOENIG A ET AL: "Object pose estimation using a set of local radiographs of a part and its CAD model" REVIEW OF PROGRESS IN QUANTITATIVE NONDESTRUCTIVE EVALUATION, REVIEW OF PROGRESS IN QUANTITATIVE NONDESTRUCTIVE EVALUATION, BRUNSWICK, ME, USA, 28 JULY-2 AUG. 1996, Seiten 829-836 vol.1, XP001069375 1997, New York, NY, USA, Plenum Press, USA ISBN: 0-306-45597-8

## Beschreibung

Die vorliegende Erfindung bezieht sich auf die Darstellung von Objekten mittels einer Durchstrahlung und auf die Rekonstruktion eines Objektes basierend auf einer solchen Darstellung des Objektes, wie es beispielsweise bei der Computertomographie der Fall ist.

Die Computertomographie ist in den 70er Jahren entwickelt worden und basiert auf einer Rekonstruktion eines Objektes basierend auf Projektionen des Objektes aus verschiedenen Durchstrahlungsrichtungen. Jede Projektionsebene gibt Auskunft über eine Absorptions- bzw. Extinktionsverteilung des Objektes quer zur Durchstrahlungsrichtung. Aus den Projektionen verschiedener Durchstrahlungsrichtungen kann dann das Objekt in Hinblick auf seine Extinktionseigenschaften bzw. seine Dichte bestimmt werden.

Bei einem Röntgencomputertomographen kreisen beispielsweise eine Röntgenröhre und ein der Röntgenröhre über das Objekt gegenüberliegender Röntgendetektor, der aus einer Zeile bzw. einem Kreissegment von Sensoren besteht, um das Objekt. Die Röntgenröhre durchstrahlt das Objekt mittels eines Strahlenfächers, wobei die das Objekt durchdringenden Strahlen von den Sensoren des Röntgendetektors empfangen werden. Dieser Vorgang wird für verschiedene Durchstrahlungsrichtungen wiederholt, wobei die Drehebene und die Ebene des Strahlenfächers stets parallel zueinander verlaufen. Bei der Rekonstruktion des Objekts anhand der erhaltenen Projektionsdaten wird ein Schnittbild durch das Objekt erzeugt. Durch relatives Verschieben des Computertomographen zu dem Objekt senkrecht zur Drehebene werden aufeinanderfolgende, benachbarte Schnittbilder erzeugt, aus denen ein dreidimensionales Bild des Objektes erzeugt werden kann. Bei moderneren Röntgencomputertomographen unit Spiralcomputertomographietechnik erfolgen die relative Verschiebung des Objektes zu dem Computertomographen und die Drehbewegung der Röntgenröhre/Röntgendetektor-Anordnung kontinuierlich, so daß die Rekonstruktion des Objekts nicht schnittbildweise sondern spiralförmig erfolgt.

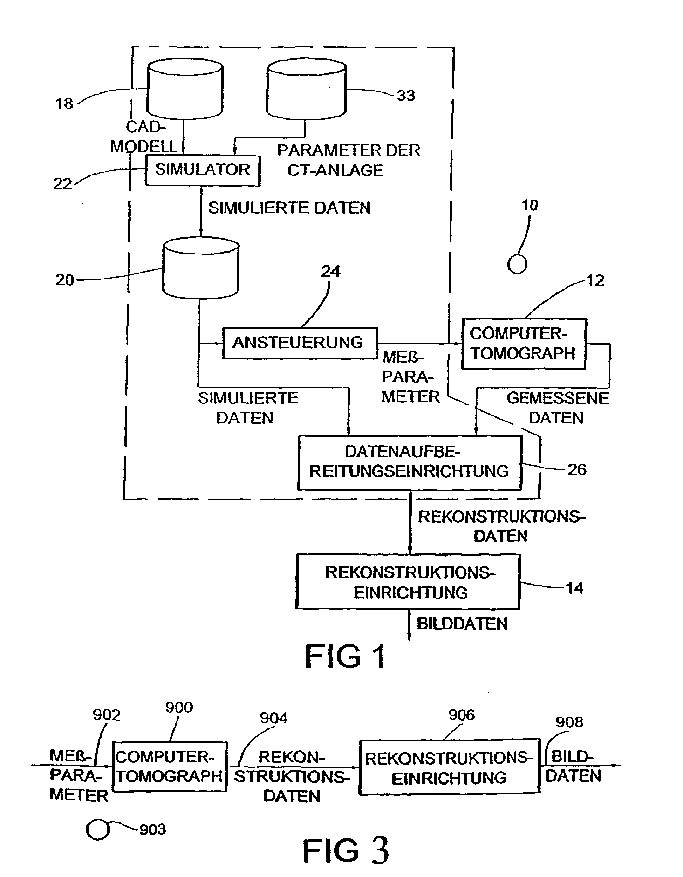

In Fig. 3 ist eine herkömmliche Anordnung zur Rekonstruktion eines Objektes basierend auf Computertomographiedaten gezeigt. Der Computertomograph 900 erhält an einem Eingang Meßparameter 902, die beispielsweise die während einer Messung eines Objekts 903, das in Fig. 3 allgemein mit einem Kreis angezeigt ist, zu verwendenden Bestrahlungsrichtungen, Intensitäten und Belichtungszeiten festlegen. Der Computertomograph mißt die Durchstrahlungen des Objektes nach Vorgabe der Meßparameter 902 und gibt die gemessenen Daten, die Projektionsdaten von Durchstrahlungen verschiedener Durchstrahlungsrichtungen enthalten, als Rekonstruktionswerte 904 an die Rekonstruktionseinrichtung 906 aus. Die Rekonstruktionseinrichtung 906 rekonstruiert aus den Rekonstruktionsdaten Bilddaten 908, die einem Bild des Objektes entsprechen, und beispielsweise Materialdichteinformationen des Objektes 903 enthalten.

Ein Problem bei der Computertomographie besteht nun darin, daß, wenn das Objekt 903, wie z.B. ein industrieller Prüfling, auch nur in einem geringen Teil der für die Rekonstruktionseinrichtung 906 zur computertomographischen Rekonstruktion notwendigen Bestrahlungsrichtungen eine Kombination aus einer hohen Absorptionsdichte einerseits und einem großen Strahlweg andererseits aufweist, wie z.B. bei Vorhandensein von stark absorbierenden Materialstegen in dem Prüfling 903, diese hohe Absorption aufgrund der begrenzten nutzbaren Detektordynamik des Computertomographen 900 häufig zu unmeßbar geringen Intensitäten und folglich zu fehler- bzw. lückenhaften Projektionsdaten in den Rekonstruktionsdaten 904 führt. Dieser fehlerhafte Anteil der Meßdaten führt bei der computertomographischen Rekonstruktion in der Rekonstruktionseinrichtung 906 zu charakteristischen Artefakten in den Bilddaten 908, die örtlich nicht auf die Gebiete hoher Dichte beschränkt sind. Bei einer Qualitätsprüfung eines Prüflings können diese Artefakte beispielsweise die Erfassung von Materialfehlern in anderen Bereichen als denjenigen hoher Absorptionsdichte verhindern. Allgemein wird aufgrund dieser Artefakte jegliche nachfolgende automatische Bildauswertung der Bilddaten 908 mit dem Ziel der Fehlererkennung wesentlich erschwert.

In der Vergangenheit wurde versucht dieses Artefaktproblem, das durch die unvollständigen bzw. fehlerhaften Rekonstruktionsdaten 904 entsteht, durch einfaches Weglassen von Rekonstruktionsdaten 904 zu umgehen, wodurch im rekonstruierten Bild 908 nichtlokale, in ihrer Form objektabhängige Artefakte, wie z.B. zylindrische Bereiche mit fehlender Materialdichteinformation, entstehen, so daß die Rekonstruktion in ihrer Gesamtheit betroffen ist. Die teilweise fehlenden Projektionsdaten mußten bei der Rekonstruktion durch spezialisierte Rekonstruktionsalgorithmen behandelt werden. Die fehlenden Bereiche in dem rekonstruierten Bild erhöhen zudem den Aufwand zur anschließenden Fehlererkennung basierend auf dem rekonstruierten Bild.

In Nuclear Instruments and Methods in Physics Research, B, September 2000, Bd. 170, Nr. 1 - 2, S. 245 - 258 wird ein Simulationsprogramm zur Simulation einer tomographischen oder radioskopischen Bilderfassung vorgestellt. Ferner werden darin mögliche Anwendungen dieses Programms beschrieben. Insbesondere werden aus der Geometrie des zu durchleuchtenden Objekts, das beispielsweise unter Verwendung von CAD-Software durch ein Dreiecksnetz beschrieben ist, durch das Programm die sich ergebenden Durchstrahlungen berechnet. Als exemplarische Anwendung wird die Verwendung der Simulation zur Optimierung experimenteller Parameter genannt. Insbesondere wird vorgeschlagen, zur Verbesserung der Erfassbarkeit von Fehlern des Objekts aus den simulierten Durchstrahlungen Signal-zu-Rausch-Verhältnis- (CNR-) Karten zu berechnen, und diese CNR-Karten zur Bestimmung optimaler Erfassungsparameter zu verwenden, die die Belichtungszeit, den Röhrenstrom und die Röhrenspannung umfassen können.

Die US 4,920,491 beschreibt ein Bildqualitätsverbesserungsschema unter Verwendung von a priori-Informationen. Insbesondere wird eine mögliche Verbesserung der Qualität von Röntgen-CT-Bildern beschrieben. Dabei werden die durch Abtastung eines Bauteils mit Röntgenstrahlen über einen begrenzten Winkelbereich gewonnenen gemessenen Projektionsdaten in dem fehlenden Winkelbereich durch berechnete Projektionsdaten ergänzt, die durch Strahlungsdämpfung aus bekannten physischen Parametern des Bauteils und der Strahlungsquelle berechnet werden. Anschließend wird ein Bild des Bauteils aus den gemessenen und berechneten Projektionsdaten rekonstruiert. Auf diese Weise können Durchstrahlungsinformationen für Richtungen gewonnen werden, bei denen das Bauteil Röntgenstrahlen zu sehr dämpft, um ein Hindurchtreten zu ermöglichen.

Die Aufgabe der vorliegenden Erfindung besteht darin, ein Verfahren und eine Vorrichtung zur Darstellung eines Objektes mittels einer Durchstrahlung zu schaffen, so daß die Darstellung besser für eine anschließende Rekonstruktion des Objektes geeignet ist, und/oder so daß die Durchstrahlung des Objekts unaufwendiger ist.

Diese Aufgabe wird durch ein Verfahren gemäß Anspruch 1 und eine Vorrichtung gemäß Anspruch 2 gelöst.

Der Erfindung liegt die Erkenntnis zugrunde, daß eine Darstellung eines Objektes mittels einer Durchstrahlung in Hinblick auf eine nachfolgende Rekonstruktion des Objekts anhand der Darstellung verbessert werden kann, indem simulierte Daten, die einer simulierten Durchstrahlung des Objektes entsprechen, bereits vor einer Rekonstruktion als Vorabinformationen zum Messen einer Durchstrahlung des Objektes und/oder zum Erzeugen der Darstellung aus einer gemessenen Durchstrahlung verwendet werden.

Ein Vorteil der vorliegenden Erfindung besteht darin, daß sie es ermöglicht, von Objekten Daten mittels Durchstrahlung so zu ermitteln, daß eine Rekonstruktion auch dort möglich ist, wo konventionelle Verfahren zu Artefakten führen.

Gemäß einem ersten Aspekt der vorliegenden Erfindung werden simulierte Daten zum Messen einer Durchstrahlung des Objektes verwendet. Gemäß einem Ausführungsbeispiel werden Vorabinformationen bzw. ein Modell des Objektes, wie z.B. ein CAD-Modell eines Sollaufbaus eines Prüflings, bereits vor der Datengewinnung bzw. der Messung des Objektes verwendet, um simulierte Durchstrahlungen des Objektes aus beispielsweise mehreren Durchstrahlungsrichtungen zu simulieren, um die simulierten Daten zu erzeugen. Diese simulierten Daten können dann dahingehend ausgewertet werden, um die Meßparameter einer Meßeinrichtung, wie z.B. eines Computertomographen, der eine Durchstrahlung des Objektes mißt, zu optimieren. So ist es beispielsweise möglich anhand der simulierten Daten eine bzw. mehrere bessere oder optimale Durchstrahlungsrichtungen und eine oder mehrere verbesserte oder optimale richtungsabhängige Intensitäten und Belichtungsdauern zu bestimmen. Die Verbesserung oder Optimierung kann beispielsweise derart durchgeführt werden, daß die bestimmten Meßparameter die Artefakte bei einer Rekonstruktion des Objekts basierend auf den gemessenen Daten möglichst gering halten. Die Meßparametereinstellung kann beispielsweise dahingehend verbessert oder optimiert werden, um die Anzahl von Durchstrahlungsrichtungen mit widrigen Durchstrahlungsbedingungen bzw. hoher Absorption möglichst gering zu halten. Ein weitere mögliche Verbesserung sieht vor, daß eine durch die Meßeinrichtung auf das Objekt ausgeübte Strahlendosis möglichst gering ist, oder daß ein optimaler Kompromiß hinsichtlich einer Aufnahmedauer und einer Detektordynamik der Meßeinrichtung getroffen wird. Folglich können durch diese Verbesserungs- bzw. Optimierungsmaßnahmen entweder die gemessenen Daten in Hinblick auf die anschließende Rekonstruktion verbessert und/oder der Durchstrahlungsaufwand verringert werden.

Gemäß einem weiteren Aspekt der vorliegenden Erfindung werden simulierte Daten, die einer simulierten Durchstrahlung des Objektes entsprechen, verwendet, um die Darstellung des Objektes aus einer gemessenen Durchstrahlung zu erzeugen. Gemäß einem Ausführungsbeispiel werden simulierte Daten, die einer simulierten Durchstrahlung des Objektes entsprechen, verwendet, um gemessene Daten, die einer gemessenen Durchstrahlung des Objektes, wie sie durch beispielsweise einen Computertomographen erzeugt wird, teilweise durch die simulierten Daten zu ergänzen und/oder zu ersetzen, und zwar noch vor einer anschließenden Rekonstruktion des Objektes basierend auf den gemessenen Daten. Beispielsweise werden bei Durchstrahlungsrichtungen, die trotz optimaler Einstellung der Aufnahmeparameter bzw. optimaler Planung der Aufnahmegeometrie bei den gemessenen Daten Bereiche sehr hoher Absorption und somit fehlerhafte Bereiche erzeugen, die aufgrund der hohen Absorption ungenauen und stark verrauschten Bereiche in den Projektionsdaten durch simulierte, beispielsweise aus einem CAD-Modell des Prüflings gewonnene Daten ersetzt. Andererseits kann eine zur Rekonstruktion erforderliche Durchstrahlungsrichtung im vorhinein durch Optimierung der Meßparameter ausgelassen werden, wobei die gemessenen Daten später um simulierte Daten, die einer simulierten Durchstrahlung in dieser Richtung entsprechen, ergänzt werden. Durch diese Verwendung der simulierten Daten zum Ergänzen und Ersetzen schlechter oder gegebenenfalls überhaupt nicht gemessener Daten vor der Rekonstruktion können Artefakte in der Rekonstruktion, die durch eine mangelnde Detektordynamik herrühren könnten, weitestgehend vermieden werden, wodurch eine Detektierbarkeit von Materialfehlern basierend auf dem rekonstruierten Bild des Objektes maximiert wird. Zudem werden Bereiche mit fehlerhafter Materialdichteinformation in den in der Rekonstruktion erzeugten Bilddaten, wie sie durch das herkömmliche Weglassen von Daten erzeugt wurden, durch das Ersetzen und Ergänzen der gemessenen Daten vermieden.

Weitere bevorzugte Ausgestaltungen der vorliegenden Erfindung sind in den beiliegenden Ansprüchen definiert.

Bevorzugte Ausführungsbeispiele der vorliegenden Erfindung werden nachfolgend bezugnehmend auf die beiliegenden Zeichnungen näher erläutert. Es zeigen:
- Fig. 1: eine Vorrichtung zur Rekonstruktion eines Objek- tes mit einem Meßsteuerungs- und Datenaufberei- tungsmodul gemäß einem Ausführungsbeispiel der vorliegenden Erfindung;
- Fig. 2: ein Flußdiagramm, das die Funktionsweise der Vor- richtung von Fig. 1 anhand von Schritten gemäß einem Ausführungsbeispiel der vorliegenden Erfin- dung veranschaulicht; und
- Fig. 3: einen herkömmlichen Aufbau zur computerto- mographischen Rekonstruktion.

Es wird darauf hingewiesen, daß sich die nachfolgende Beschreibung der vorliegenden Erfindung lediglich exemplarisch auf ein Ausführungsbeispiel bezieht, bei dem die Durchstrahlungen des Objektes mittels eines Röntgencomputertomographen durchgeführt werden, und bei dem das zu untersuchende Objekt ein Prüfling, wie z.B. eine zu untersuchende Prothese, ist. Die Erfindung ist beispielsweise auch auf andere Computertomographieverfahren anwendbar, wie z.B. die Positronenemissionstomographie (PET), oder auf andere Durchstrahlungsverfahren, bei denen Vorabinformationen bzw. Vorwissen zur Optimierung der Einstellung von Meßparametern oder zur anschließenden Ergänzung und Ersetzung der gemessenen Daten zur Verbesserung einer anschließenden Rekonstruktion verwendet werden können.

Fig. 1 zeigt den Aufbau einer Vorrichtung zur computertomographischen Rekonstruktion eines Objektes gemäß einem Ausführungsbeispiel der vorliegenden Erfindung. Gemäß dem vorliegenden Ausführungsbeispiel ist die Vorrichtung vorgesehen, um einen Prüfling 10, wie z.B. eine Prothese, beispielsweise auf eine Abweichung von einer Sollform oder auf sonstige Fehler, wie z.B. Risse oder dergleichen, hin zu überprüfen.

Die Vorrichtung von Fig. 1 umfaßt einen Computertomographen 12, eine Rekonstruktionseinrichtung 14 sowie ein Meßsteuerungs- und Datenaufbereitungsmodul 16. Das Meßsteuerungs- und Datenaufbereitungsmodul 16 ist mit einem Eingang des Computertomographen 12 verbunden, um demselben Meßparameter zuzuführen, die die Meßbedingungen während einer Messung des Computertomographen 12 an dem Objekt 10 festlegen. Das Meßsteuerungs- und Datenaufbereitungsmodul 16 ist ferner mit einem Ausgang des Computertomographen 12 verbunden, um von dem Computertomographen 12 die gemessenen Daten zu erhalten, d.h. die gemessenen Projektionsdaten des Objekts 10, die bei der Durchstrahlung des Objektes 10 unter Verwendung der festgelegten bzw. eingestellten Meßparameter, wie z.B. der eingestellten Durchstrahlungsrichtungen, Intensitäten und Belichtungszeitdauern, erhalten werden. An einem Ausgang gibt das Meßsteuerungs- und Aufbereitungsmodul 16 Rekonstruktionsdaten an die Rekonstruktionseinrichtung 14 aus, anhand derer die Rekonstruktionseinrichtung 14 Bilddaten erzeugt, die einem Bild des Objektes 10 entsprechen und beispielsweise Materialdichteinformationen über das Objekt 10 enthalten.

Das Meßsteuerungs- und Datenaufbereitungsmodul 16 umfaßt zwei Speicher 18 und 20, einen Simulator 22, eine Ansteuerung 24 und eine Datenaufbereitungseinrichtung 26. Der Simulator 22 ist derart mit den beiden Speichern 18 und 20 verbunden, daß derselbe einen Lesezugriff bezüglich des Speichers 18 und einen Schreibzugriff bezüglich des Speichers 20 aufweist. Die Ansteuerung 24 und die Datenaufbereitungseinrichtung 26 sind derart mit dem Speicher 20 verbunden, daß sie in der Lage sind, auf den Inhalt des Speichers 20 zuzugreifen. Ein Ausgang der Ansteuerung 24 ist mit einem Eingang des Computertomographen 12 verbunden, während die Datenaufbereitungseinrichtung 26 einen Eingang, der mit dem Ausgang des Computertomographen 12 verbunden ist, und einen Ausgang, der mit dem Eingang der Rekonstruktionseinrichtung 14 verbunden ist, aufweist. Auf eine Art und Weise, die im folgenden erörtert werden wird, verwenden die Ansteuerung 24 und die Meßsteuerungs- und Datenaufbereitungseinrichtung 26 die in dem Speicher 20 zur Verfügung stehenden Informationen, um optimierte Meßparameter für den Computertomographen 12 zu bestimmen und an denselben auszugeben, bzw. um gemessene Daten von dem Computertomographen 12 zu ergänzen und zu ersetzen, und als Rekonstruktionsdaten an die Rekonstruktionseinrichtung 14 auszugeben.

Der Computertomograph 12 umfaßt intern (nicht gezeigt) einen Röntgenstrahler, wie z.B. eine Röntgenröhre, der ein bestimmtes Primärröntgenspektrum aufweist, und einen Röntgendetektor, der eine bestimmte Detektorkennlinie bzw. eine frequenzabhängige Empfindlichkeit und eine bestimmte maximale Detektordynamik aufweist.

Im Anschluß an die Rekonstruktionseinrichtung 14 kann sich beispielsweise eine Qualitätsprüfungseinrichtung (nicht gezeigt) befinden, die anhand der von Rekonstruktionseinrichtung 14 erzeugten Bilddaten Fehler oder sonstige Abweichungen des Prüflings 10 von einer Sollform oder einem Sollaufbau bestimmt.

Bevor im folgenden die Funktionsweise der Vorrichtung von Fig. 1 beschrieben wird, wird darauf hingewiesen, daß die interne Aufteilung des Meßsteuerungs- und Datenaufbereitungsmoduls 16 anders sein kann als dargestellt. Insbesondere wird darauf hingewiesen, daß die einzelnen Elemente, d.h. der Simulator 22, die Ansteuerung 24, die Datenaufbereitungseinrichtung 26 und die Rekonstruktionseinrichtung 14, in Software, Firmware oder Hardware realisiert sein können. Dieselben können beispielsweise als eine integrierte Schaltung (IC = integrated circuit), eine ASIC (ASIC = application specific IC = anwendungsspezifische integrierte Schaltung), eine programmierbare Logik, ein Softwareprogramm oder eine Kombination derselben ausgebildet sein.

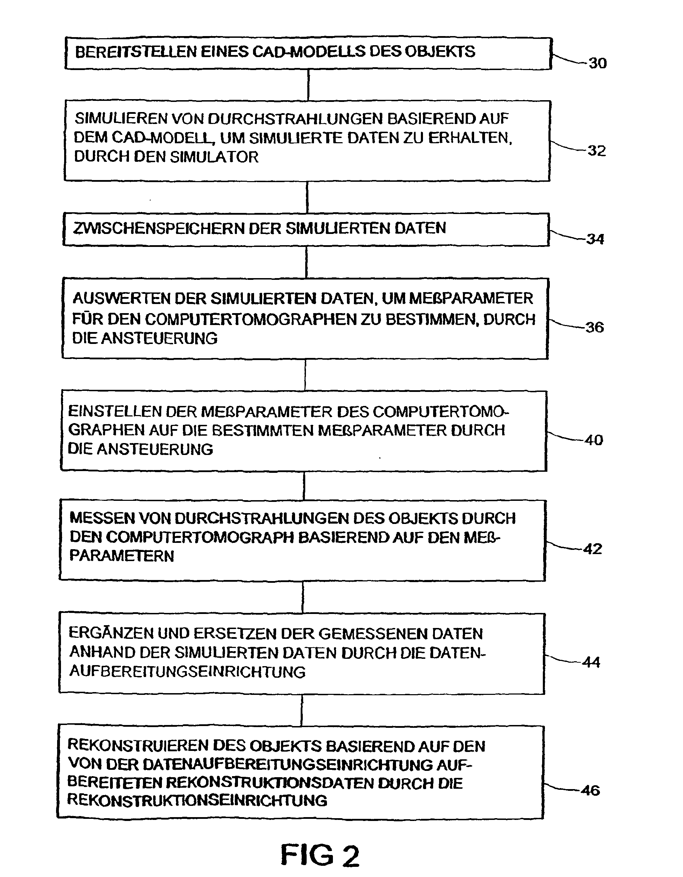

Nachdem im vorhergehenden der Aufbau der Vorrichtung von Fig. 1 beschrieben worden ist, wird im folgenden die Funktionsweise derselben anhand der in Fig. 2 gezeigten Schritte gemäß einem Ausführungsbeispiel der vorliegenden Erfindung beschrieben, wobei weiterhin Bezug auf Fig. 1 genommen wird.

In einem Schritt 30 wird zunächst ein Modell des Prüflings 10 in dem Speicher 18 bereitgestellt. Die Modelldaten des Prüflings 10 liegen beispielsweise in Form von CAD- (computer aided design = computergestützter Entwurf) Daten oder in Form von Raster- bzw. Pixeldaten, die örtliche Materialdichten und andere Materialeigenschaften angeben, vor. Die Modelldaten können dreidimensional aber auch zweidimensional sein. Insbesondere enthält das Modell des Prüflings 10 Informationen über eine Sollgeometrie und die verwendeten Materialien oder die Durchstrahlungseigenschaften des Prüflings 10. In dem Fall eines massiven Formstücks umfassen die CAD-Daten des Prüflings 10 beispielsweise lediglich eine Information über die Außenform und des verwendeten Materials des Prüflings 10. Das in dem Schritt 30 bereitgestellte Modell des Objekts 10 kann beispielsweise auch aus einer vorab angefertigten CT-Rekonstruktion entweder des Objekts 10 selbst oder eines repräsentativen Gutteils bestehen.

In einem Schritt 32 greift der Simulator 22 auf den Speicher 18 zu, um das Modell des Prüflings 10 zu erhalten, und simuliert Durchstrahlungen des Prüflings 10 basierend auf dem Modell, um simulierte Daten zu erhalten. Die Simulation wird beispielsweise unter Berücksichtigung des Extinktionsgesetzes anhand den aus den CAD-Daten in dem Speicher 18 hervorgehenden Informationen über die Extinktionseigenschaften des Objekts 10 durchgeführt. Bei dem vorliegenden Ausführungsbeispiel führt der Simulator 22 Durchstrahlungen in mehreren Durchstrahlungsrichtungen durch, so daß die simulierten Daten simulierte Projektionsdaten enthalten, die simulierten Durchstrahlungen in unterschiedlichen Durchstrahlungsrichtungen entsprechen. Bei der Simulation der Durchstrahlungen des Prüflings 10 unter den verschiedenen Projektionswinkel bzw. Durchstrahlungsrichtungen wird neben den Solldaten des zu untersuchenden Prüflings 10, die durch das CAD-Modell in dem Speicher 18 definiert werden, von der Kenntnis von beispielsweise in einem weiteren Speicher 33 bereitgestellten Parametern der CT-Anlage 12, wie z.B. des eingestrahlten Primärröntgenspektrums des Röntgenstrahlers des Computertomographen 12 und der Detektorkennlinie des Röntgendetektors des Computertomographen 12 sowie der Aufnahmegeometrie, Gebrauch gemacht.

In einem Schritt 34 werden die simulierten Daten in dem Speicher 20 zwischengespeichert, um dieselben für die Ansteuerung 24 und die Datenaufbereitungseinrichtung 26 abrufbar bereitzustellen. Die simulierten Daten werden in dem Speicher 20 beispielsweise unter Verwendung des bei der Simulation verwendeten Projektionswinkels als Index gespeichert.

In einem Schritt 36 greift die Ansteuerung 24 auf den Speicher 20 zu, um die simulierten Daten auszuwerten, um Meßparameter für den Computertomographen 12 zu bestimmen. Die Auswertung der simulierten Daten und die Bestimmung der Meßparameter für den Computertomographen 12 dienen der Planung der Aufnahme der Durchstrahlungen, die von dem Computertomographen 12 durchgeführt werden sollen, und die als Grundlage für eine anschließende computertomographische Rekonstruktion dienen, die in der Rekonstruktionseinrichtung 14 durchgeführt werden soll. Die in dem Schritt 36 bestimmten Meßparameter legen beispielsweise einen Satz von Durchstrahlungsrichtungen, Röntgenintensitäten und/oder Belichtungszeiten fest, die der Computertomograph 12 bei den Durchstrahlungen des Prüflings 10 verwenden soll. Die Bestimmung der Meßparameter kann entweder basierend auf einer Auswahl der geeigneten Aufnahmepositionen bzw. Durchstrahlungsrichtungen oder der positionsabhängigen Belichtungszeiten aus dem bei der Simulation in dem Schritt 32 verwendeten Satz von Aufnahmepositionen bzw. Belichtungszeiten durchgeführt werden.

Die Auswertung 36 der simulierten Daten, um die Meßparameter für den Computertomographen 12 zu bestimmen, kann eingestellt sein, um die Meßparameter auf verschiedene Weisen zu optimieren. Bei der Auswertung 36 werden die Meßparameter beispielsweise derart bestimmt, daß die Artefakte bei der anschließenden computertomographischen Rekonstruktion, die durch fehlende Informationen in den gemessenen Daten, verursacht durch zu hohe Objekt-Absorption bzw. mangelnde Detektordynamik, entstehen, verringert werden. Die Verringerung der Artefakte bei der anschließenden computertomographischen Rekonstruktion kann insbesondere dadurch erzielt werden, daß bei der Bestimmung der Durchstrahlungsrichtungen diejenigen Durchstrahlungsrichtungen mit besonders hoher Absorption wegen der beschränkten Dynamik des Röntgendetektors des Computertomographen 12 möglichst vermieden werden. Die Auswertung der aus dem CAD-Modell durch Simulation erhaltenen simulierten Daten kann ferner derart ausgelegt sein, daß die bestimmten Meßparameter den Aufnahmeprozeß des Computertomographen 12 derart steuern, daß die Strahlendosis, dem der Prüfling 10 während des Aufnahmeprozesses ausgesetzt ist, verringert ist. Darüber hinaus kann die Information, die aus den CAD-Modellen gewonnen wurde, zur Steuerung der Belichtungszeit bzw. der Röntgenintensität während der Computertomographieaufnahme durch den Computertomographen 12 benutzt werden, um mithin den bestmöglichen Kompromiß hinsichtlich der Aufnahmedauer und genutzter Detektordynamik zu treffen.

Die Verringerung der Artefakte bei der computertomographischen Rekonstruktion bzw. in den durch die Rekonstruktionseinrichtung 14 anschließend erzeugten Bilddaten ermöglicht gegebenenfalls eine einfachere nachfolgende automatische Bilddatenauswertung, wie z.B. eine Qualitätsprüfung, basierend auf den Bilddaten. Ferner können in dem Schritt 36 die Aufnahmen unnötiger oder aufgrund mangelnder Detektordynamik unbrauchbarer bzw. unvollständiger Projektionsdaten des zu untersuchenden Prüflings 10 bereits vor der Datenaufnahme durch den Computertomographen 12 anhand der simulierten Daten identifiziert und unterbunden bzw. ausgelassen werden.

In einem Schritt 40 stellt die Ansteuerung 24 die Meßparameter der Computertomographen 12 auf die bestimmten Meßparameter ein. Die Übertragung der Meßparameter an den Computertomographen 12 kann beispielsweise in einem Stück für alle Durchstrahlungsrichtungen erfolgen, oder erfolgt einzeln für jede Durchstrahlungsrichtung. Zudem kann die Ansteuerung analog oder digital durchgeführt werden.

In einem Schritt 42 mißt der Computertomograph 12 die Durchstrahlungen des Prüflings 10 basierend auf den Meßparametern, die derselbe von der Ansteuerung 24 empfängt. Je nach Optimierung der Auswertung der simulierten Daten in dem Schritt 36 bzw. der Bestimmung der Meßparameter ist die Strahlendosis, der der Prüfling 10 bei den Durchstrahlungen ausgesetzt ist, minimal, die Anzahl von Durchstrahlungen mit einer hohen Absorption und somit einem fehlerhaften Anteil der gemessenen Daten, die zu Artefakten bei der anschließenden Rekonstruktion führen, ist minimal, oder es ist ein bestmöglicher Kompromiß hinsichtlich Aufnahmedauer und genutzter Detektordynamik getroffen worden.

In einem Schritt 44 empfängt die Datenaufbereitungseinrichtung 26 die gemessenen Daten von dem Computertomographen 12 und ergänzt und ersetzt die gemessenen Daten anhand der in dem Speicher 20 bereitgestellten simulierten Daten. Die gemessenen Daten des Computertomographen 12 umfassen gemessene Projektionsdaten, die aus Durchstrahlungen des Prüflings 10 unter Verwendung der durch die Meßparameter festgelegten Durchstrahlungsrichtungen, positionsabhängigen Belichtungszeiten und Röntgenintensitäten erhalten wurden. Falls nun beispielsweise die Ansteuerung 24 in dem Schritt 36 bei einer bestimmten Durchstrahlungsrichtung festgestellt hat, daß dieselbe eine zu hohe Absorptionsdichte einerseits und einen zu großen Strahlweg andererseits aufweist, so daß in dieser Durchstrahlungsrichtung die Detektordynamik nicht ausreichend wäre, und deshalb in dem Schritt 36 diese Durchstrahlungsrichtung bei der Ansteuerung des Computertomographen 12 ausgelassen hat, kann die Datenaufbereitungseinrichtung 26 in dem Schritt 44 die gemessenen Daten um die fehlenden Projektionsdaten dieser Durchstrahlungsrichtung durch die entsprechenden simulierten Daten ergänzen. Für die Strahlungsrichtungen, die trotz optimaler Einstellung bzw. Planung der Aufnahmegeometrie zu gemessenen Daten geführt haben, die Bereiche sehr hoher Absorption enthalten, kann die Datenaufbereitungseinrichtung 26 in dem Schritt 44 die aus diesem Grund ungenauen und stark verrauschten Bereiche in den Projektionsdaten der gemessenen Daten durch die entsprechenden simulierten Daten ersetzen. Die so veränderten Daten gibt die Datenaufbereitungseinrichtung 26 in dem Schritt 44 an die Rekonstruktionseinrichtung 14 aus.

Die in dem Schritt 44 durchgeführte Ergänzung und Ersetzung der gemessenen Daten führt dazu, daß Artefakte bei der anschließenden Rekonstruktion durch die Rekonstruktionseinrichtung 14 weitestgehend verringert werden, und daß die Detektierbarkeit von Materialfehlern anhand der rekonstruierten Bilddaten, die von der Rekonstruktionseinrichtung 14 anschließend erzeugt werden, erhöht wird, da die Rekonstruktion weniger Fehler aufweist als bei konventionellen Verfahren.

In einem Schritt 46 empfängt die Rekonstruktionseinrichtung 14 die Rekonstruktionsdaten von der Datenaufbereitungseinrichtung 26 und führt basierend auf denselben eine Rekonstruktion des Prüflings 10 durch, und gibt die erzeugten Bilddaten aus. Die Rekonstruktion wird auf herkömmliche Weise durchgeführt, jedoch sind bei den erzeugten Bilddaten, die einem Bild des Prüflings 10 entsprechen und beispielsweise Dichte- und Materialinformationen über den Prüfling 10 enthalten, mit Hilfe des Meßsteuerungs- und Datenaufbereitungsmoduls 16 die Anzahl von Artefakten verringert, und dieselben weisen zudem keine Artefakte auf, wie es bei den auf herkömmliche Weise aus Computertomographiemeßdaten gewonnenen Bilddaten der Fall ist.

Das oben bezugnehmend auf die Fig. 1 und 2 beschriebene Ausführungsbeispiel, schafft folglich ein schnelle Aufnahmeplanung von Projektionsdaten für die Computertomographie durch geeignete Auswertung von CAD-Modell-Daten eines zu untersuchenden Objektes. Die Grundlage bildet hierbei die Einbindung der CAD-Modelle der zu untersuchenden Objekte bereits vor der Rekonstruktion bzw. vor der Datengewinnung. Unnötige oder aufgrund mangelnder Detektordynamik unbrauchbare bzw. unvollständige Projektionsdaten der zu untersuchenden Objekte können bereits vor der Datenaufnahme aus den CAD-Daten durch simulierte Röntgenprojektionen identifiziert werden und durch mit Hilfe der CAD-Modelle simulierten Daten für die Rekonstruktion ersetzt werden. Projektionswinkel, die aufgrund nicht ausreichender Detektordynamik kein auswertbares Signal erwarten lassen, werden somit durch simulierte Daten ersetzt. Für alle übrigen Positionswinkel werden dann in der Realisierung mit dem zu untersuchenden Objekt reale Röntgenprojektionen erzeugt. Anders ausgedrückt wird eine Ergänzung unvollständiger Radondatensätze unter Zuhilfenahme von CAD-Modellen erzielt. Allgemein ausgedrückt wird eine Anfertigung von simulierten Röntgendurchstrahlungen eines Prüflings vorgenommen, um das so gewonnene Wissen über Prüflinge in mehrfacher Weise für die Computertomographie einzusetzen. Als ein Ergebnis wird die Detektierbarkeit von Fehlern aufgrund der Verhinderung von nicht-lokalen Artefakten durch das Einbringen von Vorwissen über die Prüflinge erhöht.

Nachdem im vorhergehenden anhand der Fig. 1 und 2 ein Ausführungsbeispiel für den Aufbau und die Funktionsweise der Vorrichtung von Fig. 1 beschrieben worden ist, wird im folgenden auf verschiedene Alternativen hingewiesen, die gemäß der Erfindung möglich sind. Zunächst wird darauf hingewiesen, daß, wie es im vorhergehenden erwähnt worden ist, die Erfindung nicht nur auf die Röntgencomputertomographie anwendbar ist. Insbesondere ist die vorliegende Erfindung nicht auf die Art der verwendeten Strahlung begrenzt. Allgemein ist die vorliegende Erfindung auf alle Gebiete anwendbar, bei der eine Darstellung eines Objektes mittels einer Durchstrahlung durchgeführt wird.

In Hinblick auf die Schritte 30 bis 34 wird darauf hingewiesen, daß die simulierten Daten auch auf andere Weise bereitgestellt werden könnten. Der Schritt 34 kann beispielsweise fehlen, wobei statt dessen die Simulation unter Verwendung bestimmter Simulationsparameter erneut durchgeführt werden würde. Andererseits könnte zusätzlich zwischen den Schritten 36 und 40 eine Zwischenspeicherung der bestimmten Meßparameter durchgeführt werden, um eine wiederholte Auswertung der simulierten Daten in dem Fall zu vermeiden, daß mehrere Prüflinge gleicher Art überprüft werden sollen. Obwohl in dem Schritt 36 die Auswertung der simulierten Daten derart durchgeführt wird, daß die Meßparameter einen gesamten Meßvorgang des Prüflings 10 festlegen, ist es ferner möglich, daß die Schritte 36 und 40 nacheinander einzeln für beispielsweise aufeinanderfolgende Durchstrahlungsrichtungen durchgeführt werden. In diesem Fall könnten die der Auswertung in dem Schritt 36 zugrunde liegenden simulierten Daten lediglich einer simulierten Durchstrahlung in der einen Durchstrahlungsrichtung entsprechen. Die Simulation anhand des Modells könnte auch zu einem späteren Zeitpunkt durchgeführt werden. Zudem wäre es möglich, die Simulation der Modelldaten anderweitig durchzuführen bzw. durchführen zu lassen, so daß sie fehlt, und direkt anhand der simulierten Daten zu verfahren. Insbesondere kann während des Durchstrahlungsvorganges in Abhängigkeit der Simulationsergebnisse die Durchstrahlungsintensität reguliert werden, was in medizinischern Anwendungen zur Minimierung der Strahlendosis verwendet wird.

Ferner kann das Modell des Objektes auch im laufenden Betrieb dynamisch angepaßt werden. Eine initiale Vorgabe eines Modells kann somit durch einzelne Projektionen den tatsächlichen Begebenheiten angepaßt werden.

Ferner wird darauf hingewiesen, daß anders als in den Fig. 1 und 2 dargestellt entweder die Schritte 36 und 40 bzw. die Ansteuerung 24 oder der Schritt 44 bzw. die Datenaufbereitungseinrichtung 26 fehlen könnten. In dem ersteren Fall würde der Computertomograph 12 wie herkömmlich Standardmeßparameter erhalten, um die Messung der Durchstrahlungen an den Prüflingen vorzunehmen, die nicht auf irgendeine Weise anhand von simulierten Daten optimiert wurden. Die gemessenen Daten das Computertomographen würden jedoch durch die Datenaufbereitungseinrichtung 26 teilweise um die simulierten Daten ergänzt und/oder durch die simulierten Daten ersetzt werden, wodurch, wie oben beschrieben, eine Reduzierung von Artefakten in den anschließend durch die Rekonstruktionseinrichtung rekonstruierten Bilddaten auftritt. In dem anderen Fall würde der Computertomograph seine gemessenen Daten direkt an die Rekonstruktionseinrichtung 14 ausgeben. Die Meßparameter, basierend auf denen der Computertomograph die Messungen an dem Prüfling vornimmt, wären jedoch durch die Ansteuerung basierend auf den simulierten Daten optimiert, wie z.B. in Hinblick auf eine minimale Strahlendosis, eine minimale Anzahl von Artefakten bei der anschließenden Rekonstruktion oder dergleichen, wie es im vorhergehenden beschrieben wurde, so daß die gemessenen Daten anhand derer die Rekonstruktion durchgeführt wird, verbessert wäre. In beiden alternativen Fällen würde das Meßsteuerungs- bzw. Datenaufbereitungsmodul folglich eine Darstellung des Objekts erzeugen, die eine anschließende Rekonstruktion des Objekts verbessert, nämlich in dem einen Fall um simulierte Daten teilweise ergänzte bzw. durch simulierte Daten teilweise ersetzte Meßdaten bzw. Rekonstruktionsdaten und in dem anderen Fall optimierte Meßdaten, die unter optimierten Meßbedingungen gewonnen wurden, die durch unter Verwendung der Simulationsergebnisse optimierte Meßparameter festgelegt sind.

Ferner wird darauf hingewiesen, daß das vorhergehend beschriebene Ausführungsbeispiel ferner für medizinische Anwendungen anwendbar ist, um beispielsweise durch Implantate verursachte Artefakte, wie z.B. Metallartefakte, zu reduzieren. Dabei ist in dem CAD-Modell ein CAD-Modell eines Implantats mit einem geeigneten anatomischen Modell zu kombinieren. Auf gleiche Weise sind anstatt des oben beschriebenen Prüflings 10 jegliche zu untersuchenden Objekte denkbar. Insbesondere ist die vorliegende Erfindung sowohl bei der industriellen als auch der medizinischen Computertomographie anwendbar.

## Patentansprüche

1. Verfahren zur Darstellung eines Objektes (10) mittels einer zur Durchstrahlung des Objekts (10) geeigneten Meßeinrichtung (12), mit folgenden Schritten:
Bereitstellen (30, 32, 34) von simulierten Daten, die einer simulierten Durchstrahlung des Objekts (10) entsprechen; und
Verwenden (36, 40) der simulierten Daten zum Messen (42) einer Durchstrahlung des Objekts (10), wobei zur Einstellung eines Meßparameters der Meßeinrichtung (12) die simulierten Daten dahingehend ausgewertet werden, um geeignete gemessene Daten für eine tomographische Rekonstruktion des Objekts zu erhalten, **dadurch gekennzeichnet, daß** die Auswertung der simulierten Daten zur Einstellung des Meßparameters derart erfolgt, daß die Anzahl von Durchstrahlungsrichtungen mit einer hohen Absorption möglichst gering ist.

2. Vorrichtung zur Darstellung eines Objektes (10) mittels einer zur Durchstrahlung geeigneten Meßeinrichtung (12), mit
einer Einrichtung (18, 20, 22) zum Bereitstellen von simulierten Daten, die einer simulierten Durchstrahlung des Objekts (10) entsprechen; und
einer Einrichtung (24, 26) zum Verwenden der simulierten Daten zum Messen einer Durchstrahlung des Objekts (10) unter Auswertung der Simulierten zur Einstellung eines Meßparameters der Meßeinrichtung (12) dahingehend, um geeignete gemessene Daten für eine tomographische Rekonstruktion zu erhalten, **dadurch gekennzeichnet, daß** die Auswertung der simulierten Daten zur Einstellung des Parameters derart erfolgt, daß die Anzahl von Durchstrahlungsrichtungen mit einer hohen Absorption möglichst gering ist.

3. Vorrichtung gemäß Anspruch 2, bei der die Einrichtung (18, 20, 22) zum Bereistellen von simulierten Daten folgendes Merkmal aufweist:
eine Einrichtung (18) zum Bereitstellen eines Modells des Objekts (10), das Informationen über eine Geometrie und eine Durchstrahlbarkeit des Objekts (10) aufweist; und
eine Einrichtung (22) zum Simulieren einer Durchstrahlung des Objekts (10) basierend auf dem Modell des Objekts (10), um die simulierten Daten zu erhalten.

4. Vorrichtung gemäß einem der Ansprüche 2 bis 3, bei der die Einrichtung zum Verwenden der simulierten Daten ausgebildet ist, um neben einem Satz von Durchstrahlungsrichtungen auch eine Belichtungszeit und/oder eine Strahlungsintensität für die jeweiligen Durchstrahlungsrichtungen einzustellen.

5. Vorrichtung gemäß einem der Ansprüche 2 bis 4, bei der die simulierten Daten einer Mehrzahl von simulierten Durchstrahlungen des Objekts (10) mit verschiedenen Durchstrahlungsrichtungen entsprechen, die gemessenen Daten einer Mehrzahl von gemessenen Durchstrahlungen des Objekts (10) mit verschiedenen Durchstrahlungsrichtungen entsprechen, und der Meßparameter einen Satz einer Durchstrahlungsrichtung, Belichtungszeit und Strahlungsintensität für verschiedene Durchstrahlungsrichtungen aufweist.

6. Vorrichtung gemäß einem der Ansprüche 2 bis 5, bei der die Einrichtung (26) zum Verwenden folgendes Merkmal aufweist:
eine Einrichtung (26) zum Ersetzen zumindest eines Teils von gemessenen Daten, die der gemessenen Durchstrahlung entsprechen, durch einen entsprechenden Teil der simulierten Daten, um Rekonstruktionsdaten zu erhalten.

7. Vorrichtung gemäß einem der Ansprüche 2 bis 6, bei der die Einrichtung (26) zum Verwenden folgendes Merkmal aufweist:
eine Einrichtung (26) zum Ergänzen von gemessenen Daten, die der gemessenen Durchstrahlung entsprechen, durch zumindest einen Teil der simulierten Daten, um Rekonstruktionsdaten zu erhalten.

8. Vorrichtung gemäß Anspruch 6 oder 7, bei dem die gemessenen Daten einer Mehrzahl von gemessenen Durchstrahlungen entsprechen.

9. Vorrichtung gemäß einem der Ansprüche 6 bis 8, bei dem die ersetzten gemessenen Daten oder die ergänzten Rekonstruktionsdaten Durchstrahlungen entsprechen, bei denen durch das Objekt (10) eine hohe Absorption auftritt.

10. Vorrichtung gemäß einem der Ansprüche 2 bis 9, die ferner folgendes Merkmal aufweist:
eine Einrichtung zum Ausgeben entweder der gemessenen Daten oder der Rekonstruktionsdaten an eine Rekonstruktionseinrichtung (14) zum Rekonstruieren des Objekts (10) aus denselben.

11. Vorrichtung gemäß Anspruch 10, die ferner folgendes Merkmal aufweist:
eine Meßeinrichtung (12) zum Messen der Durchstrahlung des Objekts (10), um gemessene Daten zu erhalten.

12. Vorrichtung gemäß einem der Ansprüche 2 bis 11, bei der die Darstellung des Objekts (10) für eine computertomographische Auswertung geeignet ist.

13. Verfahren zum computertomographischen Rekonstruieren eines Objekts (10), mit folgenden Schritten:
Darstellen eines Objekts gemäß Anspruch 1, um eine Darstellung des Objekts (10) zu erhalten; und
Rekonstruieren (46) des Objekts (10) basierend auf der Darstellung des Objekts (10).

14. Vorrichtung zum computertomographischen Rekonstruieren eines Objekts (10), mit
einer Vorrichtung zum Darstellen eines Objekts gemäß einem der Ansprüche 2 bis 12, um eine Darstellung des Objekts (10) zu erhalten; und
einer Einrichtung (14) zum Rekonstruieren des Objekts (10) basierend auf der Darstellung des Objekts (10).

## Claims

1. Method of representing an object (10) by means of a measuring means (12) suitable for irradiation of the object (10), comprising:
providing (30, 32, 34) simulated data corresponding to a simulated irradiation of the object (10); and
using (36, 40) the simulated data for measuring (42) irradiation of the object (10), the simulated data being evaluated, for setting a measurement parameter of the measuring means (12), to obtain suitable measured data for tomographic reconstruction of the object, **characterized in that** the evaluation of the simulated data for setting the measurement parameter is effected such that the number of irradiation directions having high absorption is as small as possible.

2. Device for representing an object (10) by means of a measuring means (12) suitable for irradiation, comprising
a means (18, 20, 22) for providing simulated data corresponding to a simulated irradiation of the object (10); and
a means (24, 26) for using the simulated data for measuring irradiation of the object (10) while evaluating the simulated for setting a measurement parameter of the measuring means (12) to obtain suitable measured data for tomographic reconstruction, **characterized in that** the evaluation of the simulated data for setting the measurement parameter is effected such that the number of irradiation directions having high absorption is as small as possible.

3. Device as claimed in claim 2, wherein the means (18, 20, 22) for providing simulated data comprises:
a means (18) for providing a model of the object (10) which comprises information about a geometry and a irradiability of the object (10); and
a means (22) for simulating irradiation of the object (10) on the basis of the model of the object (10) so as to obtain the simulated data.

4. Device as claimed in any of claims 2 to 3, wherein the means for using the simulated data is configured to also adjust, in addition to a set of irradiation directions, an exposure time and/or a radiant intensity for the respective irradiation directions.

5. Device as claimed in any of claims 2 to 4, wherein the simulated data corresponds to a plurality of simulated irradiations of the object (10) with different irradiation directions, wherein the measured data corresponds to a plurality of measured irradiations of the object (10) with different irradiation directions, and wherein the measurement parameter comprises a set of a irradiation direction, exposure time and radiant intensity for various irradiation directions.

6. Device as claimed in any of claims 2 to 5, wherein the means (26) for using comprises:
a means (26) for replacing at least a portion of measured data corresponding to the measured irradiation by a corresponding portion of the simulated data so as to obtain reconstruction data.

7. Device as claimed in any of claims 2 to 6, wherein the means (26) for using comprises:
a means (26) for supplementing measured data corresponding to the measured irradiation by at least a portion of the simulated data so as to obtain reconstruction data.

8. Device as claimed in claims 6 or 7, wherein the measured data corresponds to a plurality of measured irradiations.

9. Device as claimed in any of claims 6 to 8, wherein the replaced measured data or the supplemented reconstruction data corresponds to irradiations wherein a high absorption by the object (10) occurs.

10. Device as claimed in any of claims 2 to 9, further comprising:
a means for outputting either the measured data or the reconstruction data to a reconstruction means (14) for reconstructing the object (10) from same.

11. Device as claimed in claim 10, further comprising:
a measuring means (12) for measuring the irradiation of the object (10) so as to obtain measured data.

12. Device as claimed in any of claims 2 to 11, wherein the representation of the object (10) is suitable for computer-tomographic evaluation.

13. Method for computer-tomographically reconstructing an object (10), comprising:
representing an object as claimed in claim 1 so as to obtain a representation of the object (10); and
reconstructing (46) the object (10) on the basis of the representation of the object (10).

14. Device for computer-tomographically reconstructing an object (10), comprising
a device for representing an object in accordance with any of claims 2 to 12 so as to obtain a representation of the object (10); and
a means (14) for reconstructing the object (10) on the basis of the representation of the object (10).

## Revendications

1. Procédé pour la représentation d'un objet (10) au moyen d'un moyen de mesure (12) convenant pour l'irradiation d'un objet (10), aux étapes suivantes consistant à:
préparer (30, 32, 34) des données simulées correspondant à une irradiation simulée de l'objet (10); et
utiliser (36, 40) les données simulées pour la mesure (42) d'une irradiation de l'objet (10), les données simulées étant, pour le réglage du paramètre de mesure du dispositif de mesure (12), évaluées de manière à obtenir des données mesurées appropriées pour une reconstruction tomographique de l'objet,
**caractérisé par le fait que** l'évaluation des données simulées pour un réglage du paramètre de mesure a lieu de manière que le nombre des directions d'irradiation à une absorption élevée soit aussi faible que possible.

2. Dispositif pour la représentation d'un objet (10) par un moyen de mesure (12) convenant pour l'irradiation, avec
un moyen (18, 20, 22) pour préparer des données simulées correspondant à une irradiation simulée de l'objet (10); et
un moyen (24, 26) pour utiliser des données simulées pour la mesure d'une irradiation de l'objet (10) par une évaluation des données simulées pour le réglage d'un paramètre de mesure du moyen de mesure (12) de manière à obtenir des données mesurées appropriées pour une reconstruction tomographique,
**caractérisé par le fait que** l'évaluation des données simulées pour le réglage du paramètre a lieu de manière que le nombre de directions d'irradiation à une absorption élevée soit aussi faible que possible.

3. Dispositif selon la revendication 2, dans lequel le moyen (18, 20, 22) pour préparer des données simulées présente la caractéristique suivante:
un moyen (18) pour préparer un modèle de l'objet (10) présentant des informations sur une géométrie et une irradiabilité de l'objet (10); et
un moyen (22) pour simuler une irradiation de l'objet (10) sur base du modèle de l'objet (10), pour obtenir les données simulées.

4. Dispositif selon l'une des revendications 2 à 3, dans lequel le moyen pour utiliser les données simulées est conçu pour régler, outre un ensemble de directions d'irradiation, également un temps d'illumination et/ou une intensité d'irradiation pour les directions d'irradiation respectives.

5. Dispositif selon l'une des revendications de 2 à 4, dans lequel les données simulées correspondent à une pluralité d'irradiations simulées de l'objet (10) à différentes directions d'irradiation, les données mesurées correspondent à une pluralité d'irradiations mesurées de l'objet (10) à différentes directions d'irradiation, et le paramètre de mesure présente un ensemble d'une direction d'irradiation, d'un temps d'illumination et d'une intensité d'irradiation pour différentes directions d'irradiation.

6. Dispositif selon l'une des revendications 2 à 5, dans lequel le moyen (26) pour utiliser présente la caractéristique suivante:
un moyen (26) pour substituer au moins une partie des données mesurées correspondant à l'irradiation mesurée par une partie correspondante des données simulées, pour obtenir des données de reconstruction.

7. Dispositif selon l'une des revendications 2 à 6, dans lequel le moyen (26) pour utiliser présente la caractéristique suivante:
un moyen (26) pour compléter les données mesurées correspondant à l'irradiation mesurée par au moins une partie des données simulées, pour obtenir des données de reconstruction.

8. Dispositif selon la revendication 6 ou 7, dans lequel les données mesurées correspondent à une pluralité de directions d'irradiation mesurées.

9. Dispositif selon l'une des revendications 6 à 8, dans lequel les données mesurées substituées ou les données de reconstruction complétées correspondent à des irradiations où se produit par l'objet (10) une absorption élevée.

10. Dispositif selon l'une des revendications 2 à 9, présentant par ailleurs la caractéristique suivante:
un moyen pour sortir soit les données mesurées, soit les données de reconstruction vers un moyen de reconstruction (14) pour reconstruire l'objet (10) à partir de ces dernières.

11. Dispositif selon la revendication 10, présentant par ailleurs la caractéristique suivante:
un moyen de mesure (12) pour mesurer l'irradiation de l'objet (10), pour obtenir des données mesurées

12. Dispositif selon l'une des revendications 2 à 11, dans lequel la représentation de l'objet (10) convient pour une évaluation tomographique par ordinateur.

13. Procédé de reconstruction tomographique par ordinateur d'un objet (10), aux étapes suivantes consistant à:
représenter un objet selon la revendication 1, pour obtenir une représentation de l'objet (10); et
reconstruire (46) l'objet (10) sur base de la représentation de l'objet (10).

14. Dispositif de reconstruction tomographique par ordinateur d'un objet (10), avec
un dispositif de représentation d'un objet selon l'une des revendications 2 à 12, pour obtenir une représentation de l'objet (10); et
un moyen (14) pour reconstruire l'objet (10) sur base de la représentation de l'objet (10).
